# EUROPÄISCHE PATENTSCHRIFT

(11) **EP 1 321 482 B1**
(45) Veröffentlichungstag und Bekanntmachung des Hinweises auf die Patenterteilung: **21.07.2004**
(21) Anmeldenummer: 02022725.2
(22) Anmeldetag: 11.10.2002
(51) Int. Cl.: C08G 18/80, C08F 283/01, C08G 18/63, C08F 283/02, C08F 299/04, C08G 18/79, C07D 251/34, C08G 18/10, C08G 18/42, C08G 18/62, C08G 18/48, C09D 175/06

(54) **Verwendung von Polyurethan-Pulverlacken**
Use of polyurethane powder paintings
Utilization des laques poudreuses à base des polyuréthannes

(30) Priorität: 05.12.2001 DE 10159768
(43) Veröffentlichungstag der Anmeldung: 25.06.2003
(73) Patentinhaber: Degussa AG, 40474 Düsseldorf (DE)
(72) Erfinder: Wenning, Andreas, Dr., 48301 Nottuln (DE); Grenda, Werner, 44627 Herne (DE)

(56) Entgegenhaltungen:
- EP-A- 0 512 213
- DE-A- 2 166 423
- US-A- 3 822 240
- US-A- 3 931 117
- US-A- 4 246 380
- US-A- 4 404 320
- US-A- 5 331 078
- US-A- 5 384 358

## Beschreibung

Die vorliegende Erfindung betrifft die Verwendung Pulver förmiger, Wärme härtender Lackzusammensetzungen auf Basis physikalischer Gemische einzelner Isocyanato-Isocyanurate aus aliphatischen und (cyclo)aliphatischen und/oder cycloaliphatischen Diisocyanaten und Hydroxylgruppen und Terephthalsäure haltiger Polyester für Pulver-Coil Coating-Lacke, ein Verfahren zur Herstellung von derartigen Beschichtungen sowie die mit derartigen Lacken beschichteten Metallbänder.

Hitze härtende Pulverlackzusammensetzungen werden intensiv zur Herstellung vernetzter Beschichtungen verschiedenster Substrate eingesetzt. Hitze härtende Lacke sind im Vergleich zu thermoplastischen Zusammensetzungen generell härter, resistenter gegenüber Lösemitteln und Detergentien, besitzen eine bessere Adhäsion zu metallischen Substraten und erweichen nicht bei der Exposition erhöhter Temperaturen.

Seit 1970 sind Hitze härtende Pulver förmige Massen bekannt, die man durch Reaktion eines Hydroxylgruppen haltigen Harzes mit einem maskierten Polyisocyanat erhält. Von den maskierten Polyisocyanaten haben sich als PUR-Pulverhärter ε-Caprolactam-blockierte Isophorondiisocyanataddukte durchgesetzt. Die mit diesen Härtern hergestellten PUR-Pulver werden infolge ihrer überlegenen Witterungs- und Wärmefarbstabilität zur Beschichtung verschiedenster Gegenstände aus Metall eingesetzt. Derartige Pulver werden z. B. in DE 27 35 497 beschrieben. Mit diesen Pulvern werden fertig geformte Metallteile Stück für Stück beschichtet (post coated metal).

Coil Coating hingegen ist ein Verfahren zur Beschichtung von Metallbändern mit Geschwindigkeiten von 60 bis 200 m/min. Es werden Bleche, vorzugsweise aus Stahl oder Aluminium gereinigt und mit einem Lack beschichtet. Anschließend werden diese Bleche der Weiterverarbeitung zugeführt, wo sie ihre eigentliche Form erhalten. Die wichtigsten Einsatzgebiete sind Trapezprofile, beschichtet mit Wetter beständigen Lacken, z. B. für Fassaden und Dächer sowie Türen, Fensterrahmen, Tore, Dachrinnen und Jalousien. Für den Innenbereich finden Coil Coating beschichtete Bleche für Trennwände und Deckenelemente ihre Hauptanwendung. Andere Einsatzgebiete sind aber auch Stahlmöbel, Regalbau, Ladenbau und Geräteverkleidungen. Lampen und Leuchten bilden ein weiteres wichtiges Anwendungssegment. Ebenso gibt es im Fahrzeugbereich eine breite Anwendungspalette. LKW-Aufbauten und Automobilanbauteile werden vielfach aus vorbeschichteten Materialien gefertigt.

Zum Beschichten des eingesetzten Substrats wird in der Regel eine Vorbehandlung durchgeführt. Als erste Lackschicht wird auf der späteren Sichtseite ein Primer in einer Schichtdicke von 5 bis 10 µm aufgetragen. Nach dem ersten Trockner Durchlauf erfolgt dann die eigentliche Decklackierung. Sie weist nach dem Trocknen eine Schichtdicke von etwa 20 µm auf. Teilweise wird diese Oberfläche noch mit einer temporären Schutzfolie im heißen Zustand kaschiert. Dadurch soll sie vor mechanischen Verletzungen geschützt werden. Parallel zu der Sichtseitenbeschichtung werden auch die Rückseiten mitlackiert. Als Primer werden beispielsweise Polyesterharze verwendet. Für Coil Coating beschichtete Fassaden und Dächer im korrosiven Industrieklima werden als Primer Epoxid haitige Systeme eingesetzt. In erstere Linie werden Flüssiglacke mit unzähligen Farbtönen als Decklack verwendet. Je nach Anwendungsbereich kommen z. B. Polyester-, Polyurethan- oder PVDF-Decklacke zur Anwendung. Die Schichtdicken der Decklacke liegen üblicherweise bei ca. 20 µm.

Neben den flüssigen Primern und Decklacken werden auch Pulverlacke zur Beschichtung von Metallbändern im Coil Coating-Verfahren verwendet. Pulverlacke haben gegenüber den flüssigen Lacken den großen Vorteil, dass sie lösemittelfrei und damit ökologischer sind. Allerdings war ihr Anteil an den Coil Coating-Lacksystemen bisher relativ gering.

Der Grund lag zum einen an den hohen Schichtdicken der Pulverlacke von über 40 µm. Sie führen zu optischen Defekten, da die Oberfläche nicht mehr gänzlich Poren frei ist. Durch die WO 97/47400 wurde dieser Nachteil beseitigt. Sie beschreibt ein Verfahren zur Beschichtung von Metallbändern, mit dem Pulver-Schichtdicken von weniger als 20 µm erzielt werden können.

Ein zweiter Nachteil im Vergleich zu Flüssiglacken lag in der recht langsamen Bandgeschwindigkeit beim Auftragen des Pulverlackes. Mit elektrostatischen Sprühpistolen lassen sich Metallbänder mit Pulverlack nur mit Anlagengeschwindigkeiten von maximal 20 m/min beschichten. Durch die MSC Powder Cloud™-Technologie, die z. B. von F.D. Graziano, XXIIIrd International Conference in Organic Coatings, Athen, 1997, Seiten 139 - 150 oder von M. Kretschmer, 6. DFO-Tagung Pulverlack-Praxis, Dresden, 2000, Seiten 95 - 100 beschrieben wird, lassen sich nun Bandgeschwindigkeiten von 60 bis 100 m/min realisieren.

PUR-Pulverlacke sind u.a. für ihre hohe Bewitterungsstabilität, exzellenten Verlauf und gute Flexibilität bekannt. Für den Einsatz in Coil Coating-Lacken reicht die Flexibilität der bisher bekannten Systeme jedoch oft nicht aus. Daher werden neue PUR-Pulverlacke gesucht, die der extremen Flexibilitätsanforderung von Coil Coating-Lacken genügen. Damit hätte man auch den dritten entscheidenden Nachteil gegenüber konventionellen Flüssiglacken ausgeräumt.

EP 0 047 452 beschreibt Isocyanato-Isocyanurate auf Basis von Hexamethylendiisocyanat oder Isophorondiisocyanat, die nach Blockierung der NCO-Gruppen als Vernetzer zur Herstellung flexibler Lösemittel haltiger oder Pulver förmiger Polyurethanlacke verwendet werden können.

EP 0 132 518 beschreibt ein Stoffgemisch auf Basis von Polyhydroxylkomponenten und Trimerisaten von 2-Methylpentyldiisocyanat-1,5, 2-Ethylbutandiisocyanat-1,4 und Isophorondiisocyanat, das als Bindemittel für Pulverlacke zum Beschichten Hitze härtbarer Sustrate geeignet ist.

DE 197 29 242 beschreibt Pulver förmige Bindemittel, die ein Hydroxylgruppen haltiges Polyacrylat und physikalische Gemische mindestens einer aliphatischen Isocyanurat- oder Urethan- oder Biuretgruppen haltigen Isocyanatkomponente und mindestens einer (cyclo)aliphatischen Isocyanurat- und/oder Urethangruppen haltigen isocyanatkomponente und/oder cycloaliphatischer Isocyanurat- und/oder Urethangruppen haltiger Isocyanatkomponente, deren NCO-Gruppen mit ε-Caprolactam blockiert sind, beinhalten.

Die im o. g. Stand der Technik beschriebene Pulverlacke werden ausschließlich zur Beschichtung vorgeformter Metallteile verwendet. Eine Verwendung für das Beschichten nach den Coil Coating-Verfahren wird nicht beschrieben.

Überraschenderweise wurde festgestellt, dass eine Auswahl bestimmter Vernetzer aus dem o. g. Stand der Technik mit bestimmten Hydroxylgruppen haltigen Polyestern zu Bindemitteln verarbeitet werden können, die zur Beschichtung metallischer Substrate nach dem Coil Coating-Verfahren geeignet sind.

Gegenstand der Erfindung ist somit die Verwendung von Polyurethan-Pulverlacken zur Beschichtung von Metallbändern nach dem Coil Coating-Verfahren,
dadurch gekennzeichnet,
dass diese
A) eine mit ε-Caprolactam partiell oder total blockierte Isocyanatkomponente, aus physikalischen Gemischen einzelner Isocyanato-Isocyanurate aus aliphatischen und (cyclo)aliphatischen und/oder cycloaliphatischen Diisocyanaten,
B) Hydroxylgruppen und Terephthalsäure haltige Polyester,
C) gegebenenfalls übliche Hilfs- und Zuschlagstoffe,
enthalten, und auf eine OH-Gruppe der Komponente B) 0,5 - 1,2 NCO-Gruppen der Komponente A) entfallen.

Ebenfalls Gegenstand der Erfindung ist ein Verfahren zur Beschichtung von Metallbändern nach den Coil Coating-Verfahren durch Verwendung von Polyurethan-Pulverlacken enthaltend
A) eine mit ε-Caprolactam partiell oder total blockierte Isocyanatkomponente, aus physikalischen Gemischen einzelner Isocyanato-Isocyanurate aus aliphatischen und (cyclo)aliphatischen und/oder cycloaliphatischen Diisocyanaten,
B) Hydroxylgruppen und Terephthalsäure haltige Polyester,
C) gegebenenfalls übliche Hilfs- und Zuschlagstoffe,
wobei auf eine OH-Gruppe der Komponente B) 0,5 - 1,2 NCO-Gruppen der Komponente A) entfallen.

Als Ausgangsverbindungen zur Herstellung der Isocyanatkomponente A) werden die Isocyanato-Isocyanurate von Diisocyanaten aliphatischer und (cyclo)aliphatischer und/oder cycloaliphatischer Struktur eingesetzt. Unter (cyclo)aliphatischen Diisocyanaten versteht der Fachmann hinlänglich gleichzeitig cyclisch und aliphatisch gebundene NCO-Gruppen, wie es z. B. beim Isophorondiisocyanat der Fall ist. Demgegenüber versteht man unter cycloaliphatischen Diisocyanaten solche, die nur direkt am cycloaliphatischen Ring gebundene NCO-Gruppen aufweisen. Derartige Diisocyanate werden z. B. im Houben-Weyl, Methoden der organischen Chemie, Band 14/2, S. 61 ff und J. Liebigs Annalen der Chemie, Band 562, S. 75 - 136 beschrieben. Bevorzugt werden in der Regel die technisch leicht zugänglichen aliphatischen Diisocyanate wie von Hexamethylendiisocyanat, 2-Methylpentamethylen-1,5-diisocyanat, 2-Ethyltetramethylen-1,4-diisocyanat oder Trimethylhexamethylen-1,6-diisocyanat, insbesondere das 2,2,4- und das 2,4,4-Isomere und technische Gemische beider Isomeren, die (cyclo)aliphatischen Diisocyanate wie Isophorondiisocyanat sowie die cycloaliphatischen Diisocyanate wie 4,4' Diisocyanatodicyclohexylmethan oder Norbornandiisocyanat eingesetzt.

Die Herstellung der Isocyanato-Isocyanurate erfolgte in bekannter Weise nach den Angaben der GB 13 91 066, DE 23 25 826, DE 26 44 684 oder DE 29 16 201. Die Verfahrensprodukte bestehen aus Isocyanato-Isocyanurat (Trimer) mit gegebenfalls höheren Oligomeren. Sie weisen einen Gesamt-NCO-Gehalt von 8 bis 22 Gew.-% auf.

Die erfindungsgemäß eingesetzte Isocyanatkomponente A) besteht immer aus einer physikalischen Mischung mindestens eines aliphatischen Isocyanato-Isocyanurats und mindestens einem Vertreter aus der Gruppe der (cyclo)aliphatischen und cycloaliphatischen Isocyanato-Isocyanurate.

Das Verhältnis der Isocyanato-Isocyanurate aus aliphatischen und der Summe aus (cyclo)aliphatischen und/oder cycloaliphatischen Diisocyanaten variiert bevorzugt von 70 zu 30 Gew.-% bis 30 zu 70 Gew.-%.

Zur Durchführung der Blockierungsreaktion werden im Allgemeinen die Isocyanato-Isocyanurate vorgelegt und das Blockierungsmittel ∈-Caprolactam, portionsweise zugegeben. Die Reaktion kann in Substanz oder auch in Gegenwart geeigneter (inerter) Lösemittel durchgeführt werden. Bevorzugt wird jedoch in Substanz gearbeitet. Dabei wird das Isocyanato-Isocyanurat-Gemisch auf 90 - 130 °C erhitzt. Bei dieser Temperatur erfolgt in bekannter Weise die Zugabe des Blockierungsmittels. Nach Beendigung der Zugabe des Blockierungsmittels wird die Reaktionsmischung zur Vervollständigung der Umsetzung nun etwa 1 bis 2 h bei 120 °C erhitzt. Das Blockierungsmittel wird in solchen Mengen zugegeben, dass auf 1 NCO-Äquivalent des Isocyanato-Isocyanurat-Gemisches 0,5 bis 1,1 mol Blockierungsmittel, bevorzugt 0,8 bis 1 mol, besonders bevorzugt 1 mol, zur Reaktion kommen. Zur Beschleunigung der Isocyanat-Polyadditionsreaktion können die in der Polyurethan-Chemie üblichen Katalysatoren, wie z. B. organische Zinn-, Zink- oder Aminverbindungen in einer Menge von 0,01 bis 1 Gew.-%, bezogen auf das Gesamtgemisch, zugegeben werden.

Die lösemittelfreie Blockierungsreaktion kann auch kontinuierlich im statischen Mischer oder vorteilhaft im Mehrwellenkneter, insbesondere im Zweischneckenextruder, durchgeführt werden.

Der Gesamt-NCO-Gehalt der mit ε-Caprolactam partiell oder total blockierten Isocyanatkomponente beträgt 8 bis 22 Gew.-%, vorzugsweise 9 bis 16 Gew.-%.

Zur Herstellung der erfindungsgemäß verwendeten Lackzusammensetzung werden Hydroxylgruppen und Terephthalsäure haltige Polyester verwendet.

Die einzusetzenden Hydroxylgruppen und Terephthalsäure haltigen Polyester B) weisen eine OH-Funktionalität von 2,0 bis 5, bevorzugt von 2,5 bis 4,2, ein zahlenmittleres Molekulargewicht von 800 bis 8000, bevorzugt von 1200 bis 5000, eine OH-Zahl von 20 bis 200 mg KOH/g, bevorzugt von 30 bis 100 mg KOH/g, eine Viskosität bei 160 °C von < 80 000 mPa·s, bevorzugt von < 60 000 mPa·s und einen Schmelzpunkt von ≥ 70 °C bis ≤ 120 °C, bevorzugt von ≥ 75 bis ≤ 100 °C, auf. Wesentlich ist, dass Terephthalsäure und/oder Ester der Terephthalsäure zumindest anteilig mitverwendet wird.

Der Anteil an der erfindungswesentlich verwendeten Terephthalsäure kann je nach Verwendungszweck stark variieren, so dass dieser 1 - 100 mol-%, bevorzugt 5 - 100 mol-%, bezogen auf alle Carbonsäuren oder deren Ester oder Anhydride, die zur Herstellung des Polyesters B) eingesetzt werden, betragen kann.

Die Polyester können aus an sich bekannte Weise durch Kondensation von Polyolen und Polycarbonsäuren in einer Inertgasatmosphäre bei Temperaturen von 100 bis 260 °C, vorzugsweise von 130 bis 220 °C, in der Schmelze oder in azeotroper Fahrweise gewonnen werden, wie es z. B. in Methoden der Organischen Chemie (Houben-Weyl), Bd. 14/2, 1 - 5, 21 - 23, 40 - 44, Georg Thieme Verlag, Stuttgart, 1963, bei C. R. Martens, Alkyd Resins, 51 - 59, Reinhold Plastics Appl., Series, Reinhold Publishing Comp., New York, 1961 oder in DE 27 35 497 und DE 30 04 903, beschrieben ist.

Das Mischungsverhältnis von Hydroxylgruppen und Terephthalsäure haltigen Polyestern und blockierter Isocyanatkomponente wird in der Regel so gewählt, dass auf eine OH-Gruppe 0,5 bis 1,2, bevorzugt 0,8 bis 1,1, ganz besonders bevorzugt 1,0 NCO-Gruppen entfallen.

Die Isocyanatkomponente A) wird für die Herstellung von PUR-Pulverlacken mit dem geeigneten Hydroxylgruppen und Terephthalsäure haltigen Polyester B) und ggf. üblichen Hilfs- und Zuschlagstoffen C) gemischt. Als Hilfs- und Zuschlagstoffe C) können z. B. Katalysatoren, Pigmente, Füllstoffe, Farbstoffe, Verlaufmittel, z. B. Siliconöl und flüssigen Acrylatharzen, Licht- und Hitzestabilisatoren, Antioxidantien, Glanzverbesserer oder Effektadditive eingesetzt werden. Die Komponenten A), B) und C) werden in der Schmelze homogenisiert. Dies kann in geeigneten Apparaten, z. B. in beheizbaren Knetern, vorzugsweise aber durch Extrudieren erfolgen, wobei Temperaturgrenzen von 130 bis 140 °C nicht überschritten werden sollten. Die extrudierte homogenisierte Masse wird nach Abkühlen auf Raumtemperatur und nach geeigneter Zerkleinerung zum sprühfertigen Pulver vermahlen. Das Auftragen des sprühfertigen Pulvers auf geeignete Substrate kann nach den bekannten Verfahren, z. B. elektrostatisches Pulversprühen, Wirbelsintern oder elektrostatisches Wirbelsintern erfolgen. Nach dem Pulverauftrag werden die beschichteten Werkstücke zur konventionellen Aushärtung in einem Ofen 60 Minuten bis 30 Sekunden auf eine Temperatur von 160 bis 250 °C, vorzugsweise 30 bis 1 Minuten bei 170 bis 240 °C erhitzt. Bei der Verwendung eines Coil Coating Ofens betragen die Aushärtebedingungen gewöhnlich 90 bis 10 s bei Temperaturen von 200 bis 350 °C.

Um die Geliergeschwindigkeit der Wärme härtbaren Pulverlacke zu erhöhen, können Katalysatoren zugesetzt werden. Als Katalysatoren verwendet man z. B. Organozinnverbindungen wie Dibutylzinndilaurat, Zinn(II)octoat, Dibutylzinnmaleat oder Butylzinn-tris(2-ethylhexanoat). Die Menge an zugesetztem Katalysator beträgt 0,01 bis 1,0 Gew.-%, bezogen auf die gesamte Pulverlackmenge.

Mit der erfindungsgemäß verwendeten Lackzusammensetzung lassen sich äußerst flexible, überbrennbare und witterungsbeständige PUR-Pulverlacke und Pulver Coil Coating-Lacke herstellen.

Nachfolgend wird der Gegenstand der Erfindung anhand von Beispielen erläutert.

### Beispiele

### A) Herstellung ε-Caprolactam blockierter Isocyanatkomponenten

### Beispiel 1

699,8 g Desmodur N 3300 (Polyisocyanato-Isocyanurat auf Basis Hexamethylendiisocyanat der Fa. Bayer) und 1632,8 g VESTANAT T 1890 (Polyisocyanato-Isocyanurat auf Basis Isophorondiisocyanat der Fa. Degussa) wurden auf 100°C aufgeheizt. Es wurden 3,5 g Dibutylzinndilaurat zugegeben. Danach wurden portionsweise 1163,9 g ε-Caprolactam zugegeben. Eine Stunde nach der letzten Portion an ε-Caprolactam war die Reaktion beendet. Danach wurde das Reaktionsgemisch auf Raumtemperatur abgekühlt. Das Reaktionsprodukt hatte einen Gehalt an freien NCO-Gruppen von 0,4 %, einen NCO-Gesamt-Gehalt von 12,0 % und einen Schmelzbereich von 88 - 91 °C.

### Beispiel 2 (Vergleich)

488,4 g Isophorondiisocyanat wurde unter Rühren auf 110 °C aufgeheizt und 68,3 g Monoethylenglykol zudosiert. Nach einer Reaktionszeit von 60 Minuten wurden 249,1 g ε-Caprolactam zugegeben. Nach wiederum 60 Minuten wurde das Produkt abgekühlt und zerkleinert. Das Reaktionsprodukt hatte einen Gehalt an freien NCO-Gruppen von 0,2 %, einen Gehalt an blockierten NCO-Gruppen von 11,4 % und einen Schmelzbereich von 65 - 75 °C.

### B) Polyester

Es wurde ein Polyester folgender Zusammensetzung eingesetzt: als Säurekomponente: 100 mol-% Dimethylterephthalat; als Alkoholkomponenten: 57,5 mol-% Monoethylengykol, 0,5 mol-% Hexandiol-1,6, 38,5 mol-% Neopentylglykol und 3,5 mol-% Glyzerin. Der Polyester hatte eine OH-Zahl von 39 mg KOH/g, eine Säurezahl von 2,5 mg KOH/g und eine Glasübergangstemperatur von 60 °C.

### C) Polyurethan-Pulverlacke

### Allgemeine Herstellungsvorschrift

Die zerkleinerten Produkte - blockiertes Polyisocyanat (Vernetzer), Polyester, Verlaufmittel, Entgasungsmittel und Katalysator-Masterbatch - werden mit dem Weißpigment in einem Kollergang innig vermischt und anschließend im Extruder bis maximal 130 °C homogenisiert. Nach dem Erkalten wird das Extrudat gebrochen und mit einer Stiftmühle auf eine Korngröße < 100 µm gemahlen. Das so hergestellte Pulver wird mit einer elektrostatischen Pulverspritzanlage bei 60 kV auf entfettete, Eisen phosphatierte Stahlbleche appliziert und in einem Umlufttrockenschrank bzw. in einem Coil Coating Ofen eingebrannt.

Die Formulierungen enthielten 30 Gew.-% Kronos 2160 (Titandioxid der Fa. Kronos), 1Gew.-% Resiflow PV 88 (Verlaufmittel der Worlée-Chemie), 0,5 Gew.-% Benzoin (Entgasungsmittel der Fa. Merck-Schuchard) und 0,1 Gew.-% Dibutylzinndilaurat (Katalysator der Fa. Crompton Vinyl Additives GmbH). Das OH/NCO-Verhältnis betrug 1 : 1.

**Tabelle 1:**

| Lackdaten weißpigmentierter PUR-Pulverlacke | | | | |
|---|---|---|---|---|
| Vernetzer/Polyester | A 1 / B 1 | | A 2 / B 1 (Vergleich) | |
| Einbrennbedingung | 200 °C/10 min | 241°C/70 sec | 200 °C/10 min | 241 °C/70 sec |
| Schichtdicke (µm) | 55 - 65 | 31 - 44 | 58 - 65 | 32 - 44 |
| Glanz 60°-W. | 91 | 90 | 87 | 74 - 83 |
| Tiefung (mm) | > 10 | - | > 10 | - |
| KS dir./indir. (inch 1b) | > 160 / > 160 | - | 80/20 | - |
| T-Bend | - | 0 T | - | 1,5 T |

Die Abkürzungen in der Tabelle bedeuten:

| | |
|---|---|
| Glanz 60°-W. | = Messung des Glanzes nach Gardner (ASTM-D 5233) |
| Tiefung | = Tiefung nach Erichsen (DIN 53 156) |
| KS dir./indir. | = direkter und indirekter Kugelschlag (ASTM D 2794 - 93) |
| T-Bend | = Verfonnungstest (ECCA T 7) |

## Patentansprüche

1. Verwendung von Polyurethan-Pulverlacken zur Beschichtung von Metallbändern nach
dem Coil Coating-Verfahren,
**dadurch gekennzeichnet,**
**dass** diese
A) eine mit ε-Caprolactam partiell oder total blockierte Isocyanatkomponente, aus physikalischen Gemischen einzelner Isocyanato-Isocyanurate aus aliphatischen und (cyclo)aliphatischen und/oder cycloaliphatischen Diisocyanaten,
B) Hydroxylgruppen und Terephthalsäure haltige Polyester,
C) gegebenenfalls übliche Hilfs- und Zuschlagstoffe,
enthalten, und auf eine OH-Gruppe der Komponente B) 0,5 - 1,2 NCO-Gruppen der Komponente A) entfallen.

2. Verwendung nach Anspruch 1,
**dadurch gekennzeichnet,**
**dass** als Ausgangsverbindungen für die Isocyanatkomponente A) die Isocyanato-Isocyanurate ausgewählt aus Hexamethylendiisocyanat, 2-Methylpentamethylen-1,5-diisocyanat, 2-Ethyltetramethylen-1,4-diisocyanat, Tri-methylhexamethylen-1,6-diisocyanat, Isophorondiisocyanat, 4,4'-Diisocyanatodicyclohexylmethan oder Norbornandiisocyanat eingesetzt werden.

3. Verwendung nach mindestens einem der Ansprüche 1 bis 2,
**dadurch gekennzeichnet,**
**dass** das Verhältnis der Isocyanato-Isocyanurate aus aliphatischen und der Summe aus (cyclo)aliphatischen und/oder cycloaliphatischen Diisocyanaten 70 zu 30 Gew.-% bis 30 zu 70 Gew.-% beträgt.

4. Verwendung nach Anspruch 3,
**dadurch gekennzeichnet,**
**dass** als aliphatische Diiisocyanate Hexamethylendiisocyanat, 2-Methylpentamethylen-1,5-diisocyanat, 2-Ethyltetramethylen-1,4-butandiisocyanat oder Trimethylhexamethylen-1,6-diisocyanat, als (cyclo)alilphatisches Düsocyanat Isophorondiisocyanat und als cycloaliphatische Diisocyanate 4,4'-Diisocyanato-cyclohexylmethan oder Norbornandiisocyanat eingesetzt werden.

5. Verwendung nach mindestens einem der Ansprüche 1 bis 4,
**dadurch gekennzeichnet,**
**dass** die Isocyanatkomponente einen Gesamt-NCO-Gehalt von 8 bis 22 Gew.-% aufweist.

6. Verwendung nach mindestens einem der Ansprüche 1 bis 5,
**dadurch gekennzeichnet,**
**dass** die Isocyanatkomponente so blockiert vorliegt, dass auf ein NCO-Äquivalent 0,5 - 1,1 mol ε-Caprolactam kommen.

7. Verwendung nach mindestens einem der Ansprüche 1 bis 6,
**dadurch gekennzeichnet,**
**dass** die Hydroxylgruppen und Terephthalsäure haltigen Polyester B) eine Funktionalität von 2,0 bis 5, eine OH-Zahl von 20 bis 200 mg KOH/g, eine Viskosität bei 160 °C von < 60 000 mPa·s und einen Schmelzpunkt ≥ 70 °C bis ≤ 120 °C aufweisen.

8. Verwendung nach mindestens einem der Ansprüche 1 bis 7,
**dadurch gekennzeichnet,**
**dass** der Anteil an Terephthalsäure im Polyester B) von 1 - 100 mol-%, bezogen auf alle zur Herstellung des Polyesters B) eingesetzten Carbonsäuren oder deren Ester oder Anhydride, beträgt.

9. Verwendung nach mindestens einem der Ansprüche 1 bis 8,
**dadurch gekennzeichnet,**
**dass** ein OH/NCO-Verhältnis von 1 : 0,5 bis 1,2 zugrunde liegt.

10. Verwendung nach mindestens einem der Ansprüche 1 bis 9,
**dadurch gekennzeichnet,**
**dass** ein OH/NCO-Verhältnis von 1 : 0,8 bis 1,1 zugrunde liegt.

11. Verwendung nach mindestens einem der Ansprüche 1 bis 10,
**dadurch gekennzeichnet,**
**dass** ein OH/NCO-Verhältnis von 1 : 1 zugrunde liegt.

12. Verwendung nach mindestens einem der Ansprüche 1 bis 11,
**dadurch gekennzeichnet,**
**dass** die PUR-Pulverlacke Katalysatoren in einer Konzentration von 0,01 bis 1,0 Gew.-%, bezogen auf die gesamte Pulverlackmenge, enthalten.

13. Verwendung nach Anspruch 12,
**dadurch gekennzeichnet,**
**dass** die PUR-Pulverlacke organische Zinnverbindungen in einer Konzentration von 0,01 bis 1,0 Gew.-%, bezogen auf die gesamte Pulverlackmenge, enthalten.

14. Verwendung nach mindestens einem der Ansprüche 1 bis 13,
**dadurch gekennzeichnet,**
**dass** als Komponente C) Katalysatoren, Pigmente, Füllstoffe, Farbstoffe, Verlaufmittel, Licht- und Hitzestabilisatoren, Antioxidantien, Glanzverbesserer und/oder Effektadditive enthalten sind.

15. Verfahren zur Beschichtung von Metallbändern nach den Coil Coating-Verfahren durch Verwendung von Polyurethan-Pulverlacken enthaltend
A) eine mit ε-Caprolactam partiell oder total blockierte Isocyanatkomponente, aus physikalischen Gemischen einzelner Isocyanato-Isocyanurate aus aliphatischen und (cyclo)aliphatischen und/oder cycloaliphatischen Diisocyanaten,
B) Hydroxylgruppen und Terephthalsäure haltige Polyester,
C) gegebenenfalls übliche Hilfs- und Zuschlagstoffe,
wobei auf eine OH-Gruppe der Komponente B) 0,5 - 1,2 NCO-Gruppen der Komponente A) entfallen.

16. Verfahren nach Anspruch 15,
**dadurch gekennzeichnet,**
**dass** die Polyurethanlacke Verbindungen nach mindestens einem der Ansprüche 2 bis 14 als Ausgangskomponente enthalten.

## Claims

1. The use of a polyurethane powder coating material for coating metal coils by the coil coating process, **characterized in that** said material comprises
(A) an isocyanate component which is totally or partly blocked with ε-caprolactam and comprises physical mixtures of individual isocyanato isocyanurates of aliphatic and (cyclo) aliphatic and/or cycloaliphatic diisocyanates,
(B) polyesters containing hydroxyl groups and terephthalic acid, and
(C) if desired, customary auxiliaries and additives,
and there are 0.5-1.2 NCO groups of component A) per OH group of component B).

2. The use according to claim 1, **characterized in that** the isocyanato isocyanurates of diisocyanates selected from hexamethylene diisocyanate, 2-methylpentamethylene 1,5-diisocyanate, 2-ethyltetramethylene 1,4-diisocyanate, trimethylhexamethylene 1,6-diisocyanate, isophorone diisocyanate, 4,4'-diisocyanatodicyclohexylmethane, and norbornane diisocyanate are used as starting compounds for the isocyanate component A).

3. The use according to at least one of claims 1 and 2, **characterized in that** the ratio of the isocyanato isocyanurates of aliphatic diisocyanates to the sum of (cyclo)aliphatic and/or cycloaliphatic diisocyanates is from 70:30% by weight to 30:70% by weight

4. The use according to claim 3, **characterized in that** hexamethylene diisocyanate, 2-methylpentamethylene 1,5-diisocyanate, 2-ethyltetramethylene 1,4 diisocyanate or trimethylhexamethylene 1,6-diisocyanate are used as aliphatic diisocyanates, isophorone diisocyanate is used as (cyclo)aliphatic diisocyanate and 4,4'-diisocyanatocyclohexylmethane or norbornane diisocyanate are used as cycloaliphatic diisocyanate.

5. The use according to at least one of claims 1 to 4, **characterized in that** the isocyanate component has a total NCO content of from 8 to 22% by weight.

6. The use according to at least one of claims 1 to 5, **characterized in that** the isocyanate component is blocked such that there are 0.5 - 1.1 mol of ε-caprolactam per NCO equivalent

7. The use according to at least one of claims 1 to 6, **characterized in that** the polyesters B) containing hydroxyl groups and terephthalic acid have a functionality of from 2.0 to 5, an OH number of from 20 to 200 mg KOH/g, a viscosity at 160°C of < 60 000 mPa·s, and a melting point ≥ 70°C to ≤ 120°C.

8. The use according to at least one of claims 1 to 7, **characterized in that** the proportion of terephthalic acid in the polyesters B) is 1 - 100 mol%, based on all of the carboxylic acids or their esters or anhydrides used for preparing the polyesters B).

9. The use according to at least one of claims 1 to 8, **characterized in that** the underlying OH/NCO ratio is 1: from 0.5 to 1.2.

10. The use according to at least one of claims 1 to 9, **characterized in that** the underlying OH/NCO ratio is 1: from 0.8 to 1.1.

11. The use according to at least one of claims 1 to 10, **characterized in that** the underlying OH/ NCO ratio is 1:1.

12. The use according to at least one of claims 1 to 11, **characterized in that** the PU powder coating material contains catalyst in a concentration of from 0.01 to 1.0% by weight, based on the total amount of powder coating material.

13. The use according to claim 12, **characterized in that** the PU powder coating material contains organotin compound in a concentration of from 0.01 to 1.0% by weight, based on the total amount of powder coating material.

14. The use according to at least one of claims 1 to 13, **characterised in that** catalyst, pigment, filler, dye, levelling agent, light stabilizer, heat stabilizer, antioxidant, gloss enhancer and/or effect additive are present as component C).

15. A process for coating metal coils by the coil coating process by using a polyurethane powder coating material comprising
(A) an isocyanate component which is totally or partly blocked with ε-caprolactam and comprises physical mixtures of individual isocyanato isocyanurates of aliphatic and (cyclo) aliphatic and/or cycloaliphatic diisocyanates,
(B) polyesters containing hydroxyl groups and terephthalic acid, and
(C) if desired, customary auxiliaries and additives,
and there are 0.5 - 1.2 NCO groups of component A) per OH group of component B).

16. A process according to claim 15, **characterized in that** the polyurethane coating material comprises compounds as set forth in at least one of claims 2 to 14 as starting components.

## Revendications

1. Utilisation de peintures en poudre à base de polyuréthane pour le revêtement de bandes métalliques selon le procédé de prélaquage en continu,
**caractérisée en ce que**
celles-ci contiennent
A) un composant isocyanate bloqué totalement ou partiellement avec du ε-caprolactame, obtenu à partir d'un mélange physique de différents isocyanato-isocyanurates de diisocyanates aliphatiques et (cyclo)aliphatiques et/ou cycloaliphatiques,
B) des polyesters contenant de l'acide téréphtalique et des groupes hydroxyle,
C) le cas échéant, des additifs et adjuvants traditionnels, et
0,5 à 1,2 groupes NCO des composés A) reviennent à un groupe OH du composé B).

2. Utilisation selon la revendication 1,
**caractérisée en ce que**
comme composés de départ pour les composants isocyanates A), les isocyanato-isocyanurates sélectionnés parmi le diisocyanate d'hexaméthylène, le 2-méthylpentaméthylène-1,5-düsocyanate, le 2-éthyltétraméthylène-1,4-diisocyanate, le tri-méthylhexaméthylène-1,6-diisocyanate, le diisocyanate d'isophorone, le 4,4'-diisocyanatodicyclohexylméthane ou le diisocyanate de norbornane sont utilisés.

3. Utilisation selon au moins l'une quelconque des revendications 1 à 2,
**caractérisée en ce que**
le rapport de l'isocyanato-isocyanurate à partir des diisocyanates aliphatiques et de la somme des diisocyanates (cyclo)aliphatiques et/ou cycloaliphatiques est de 70 à 30 % en poids jusqu'à 30 à 70 % en poids.

4. Utilisation selon la revendication 3,
**caractérisée en ce que**
comme diisocyanates aliphatiques, on utilise le diisocyanate d'hexaméthylène, le 2-méthylpentaméthylène-1,5-diisocyanate, le 2-éthyltétraméthylène-1,4-butanediisocyanate ou le triméthylhexaméthylène-1,6-diisocyanate, comme diisocyanate (cyclo)aliphatique le diisocyanate d'isophorone et comme diisocyanates cycloaliphatiques le 4,4'-diisocyanato-cyclohexylméthane ou le diisocyanate de norbornane.

5. Utilisation selon au moins l'une quelconque des revendications 1 à 4,
**caractérisée en ce que**
le composant isocyanate présente une teneur NCO totale de 8 à 22 % en poids.

6. Utilisation selon au moins l'une quelconque des revendications 1 à 5,
**caractérisée en ce que**
le composant isocyanate se présente de façon bloquée de sorte que le ε-caprolactame atteigne un équivalent NCO de 0,5 à 1,1 % en moles.

7. Utilisation selon au moins l'une quelconque des revendications 1 à 6,
**caractérisée en ce que**
les polyesters B) contenant des groupes hydroxyle et des acides téréphtaliques présentent une fonctionnalité de 2,0 à 5, une quantité OH de 20 à 200 mg KOH/g, une viscosité à 160°C de < 60 000 mPa•s et un point de fusion de ≥ 70°C à ≤ 120°C.

8. Utilisation selon au moins l'une quelconque des revendications 1 à 7,
**caractérisée en ce que**
la proportion d'acide téréphtalique dans le polyester B) représente de 1 à 100 % en moles par rapport à l'ensemble des acides carboniques utilisé pour la fabrication des polyesters B) ou de leurs esters ou anhydres.

9. Utilisation selon au moins l'une quelconque des revendications 1 à 8,
**caractérisée en ce que**
un rapport OH/NCO se situe entre 1 : 0,5 et 1,2.

10. Utilisation selon au moins l'une quelconque des revendications 1 à 9,
**caractérisée en ce que**
un rapport OH/NCO se situe entre 1 : 0,8 et 1,1.

11. Utilisation selon au moins l'une quelconque des revendications 1 à 10,
**caractérisée en ce que**
le rapport OH/NCO se situe à 1 : 1.

12. Utilisation selon au moins l'une quelconque des revendications 1 à 11,
**caractérisée en ce que**
les catalyseurs de peintures en poudre à base de PUR sont contenus selon une concentration de 0,01 à 1,0 % en poids par rapport à la quantité de peintures en poudre totale.

13. Utilisation selon la revendication 12,
**caractérisée en ce que**
la peinture en poudre à base de PUR contient des composés organiques d'étain selon une concentration de 0,01 à 1,0 % en poids par rapport à la quantité de peintures en poudre totale.

14. Utilisation selon au moins l'une quelconque des revendications 1 à 13,
**caractérisée en ce que**
comme composants C) sont contenus des catalyseurs, des pigments, des agents de remplissage, des colorants, des produits nivelant les inégalités, des stabilisateurs de lumière et thermiques, des antioxydants, des agents d'amélioration de brillance et des additifs d'effet.

15. Procédé pour le revêtement de bandes métalliques selon le procédé de prélaquage en continu par l'utilisation de peintures en poudre de polyuréthane contenant
A) un composant isocyanate bloqué totalement ou partiellement avec du ε-caprolactame, obtenu à partir d'un mélange physique de différents isocyanato-isocyanurates de diisocyanates aliphatiques et (cyclo)aliphatiques et/ou cycloaliphatiques,
B) des polyesters contenant de l'acide téréphtalique et des groupes hydroxyle,
C) le cas échéant, des additifs et adjuvants traditionnels, et
0,5 à 1,2 groupes NCO des composés A) reviennent à un groupe OH du composé B).

16. Procédé selon la revendication 15,
**caractérisé en ce que**
les peintures à base de polyuréthane comprennent des composés selon au moins l'une quelconque des revendications 2 à 14 comme composants de départ.
